# EUROPÄISCHE PATENTANMELDUNG

(11) **EP 2 959 926 A1**
(43) Veröffentlichungstag der Anmeldung: **30.12.2015**
(21) Anmeldenummer: 14174595.0
(22) Anmeldetag: 26.06.2014
(51) Int. Cl.: A61M 1/00

(54) **Medizinische Saugpumpe und Fluidsammelbehälter**

(71) Anmelder: Medela Holding AG, 6340 Baar (CH)
(72) Erfinder: Giezendanner, Charles, 6443 Morschach (CH); Melzer, Martin, 6330 Cham (CH); Walti, Martin, 8038 Zürich (CH)
(74) Vertreter: Clerc, Natalia

(57) **Zusammenfassung**

Eine medizinische Saugpumpe zum Absaugen von Körperfluid weist ein Saugpumpengehäuse (1), einen lösbar am Gehäuse (1) gehaltenen Fluidsammelbehälter (2, 4) sowie mindestens einen Füllstandsensor (30, 31) zur Detektion eines Füllstandes des Fluidsammelbehälters (2, 4) auf. Der Fluidsammelbehälter (2) weist eine erste Seitenwand (20, 60) und mindestens eine weitere Seitenwand (21, 22, 23; 61, 49, 63) auf, welche gemeinsam mindestens einen Innenraum zur Aufnahme des abgesaugten Fluids begrenzen, wobei der Innenraum eine mit Fluid befüllbare Höhe aufweist. Der mindestens eine Füllstandsensor (30, 31) erstreckt sich mindestens annähernd parallel zur ersten Seitenwand (20, 60) über mindestens einen Teilbereich der befüllbaren Höhe. Die erste Seitenwand (20, 60) ist mindestens abschnittsweise plan ausgebildet und der mindestens eine Innenraum verjüngt sich mindestens über einen Teilbereich der befüllbare Höhe im Querschnitt in vertikaler Richtung von oben nach unten.

## Beschreibung

### TECHNISCHES GEBIET

Die vorliegende Erfindung betrifft eine medizinische Saugpumpe sowie einen Fluidsammelbehälter einer derartigen Saugpumpe.

### STAND DER TECHNIK

Medizinische Saugpumpen zum Absaugen von Körperfluid werden in verschiedenen Bereichen eingesetzt; beispielsweise bei oder nach chirurgischen Eingriffen, in der Wunddrainage, der Thoraxdrainage oder beim Absaugen von Körperfetten. Diese Vakuumpumpsysteme weisen üblicherweise eine Vakuumpumpe, einen oder mehrere Fluidsammelbehälter und eine Drainageschlauchverbindung zwischen Patient und Fluidsammelbehälter auf.

Mittels einer Vakuumpumpe wird ein Unterdruck im Fluidsammelbehälter erzeugt, wodurch sich das Fluid oder Sekret von einer Kavität des Patienten über den Drainageschlauch in den Behälter saugen und dort sammeln lässt. WO 2007/128156 und WO 2008/141471 offenbaren derartige medizinische Saugpumpen, bei welchen der Fluidsammelbehälter schwenkbar und lösbar an einem Pumpengehäuse befestigt ist. WO 2011/054118 und WO 2012/097462 schlagen vor, einen kapazitiven Füllstandsensor am Gehäuse anzuordnen, um den Füllstand im Fluidsammelbehälter zu bestimmen. Diese Füllstandsensoren haben sich zwar bewährt. Es ist jedoch kaum möglich, kleine Füllstandmengen zu bestimmen.

Medizinische Saugpumpen und Fluidsammelbehälter werden auch in der maschinellen Autotransfusion, insbesondere für Herz-Lungen-Maschinen, verwendet.

EP 1 589 325 und WO 2009/098077 offenbaren zudem offene Krüge, bei welchen ein Flüssigkeitskonsum festgestellt werden kann. Hierfür sind die Krüge in Haltern angeordnet, welche mit Füllstandsensoren versehen sind. Die Krüge weisen eine konische Grundform auf und entsprechend sind ihre Halter ausgebildet. Die Füllstandsensoren erstrecken sich entlang einer schrägen Wand des Halters.

### DARSTELLUNG DER ERFINDUNG

Es ist eine Aufgabe der Erfindung, eine medizinische Saugpumpe und einen zugehörigen Fluidsammelbehälter zu schaffen, bei welchen mit einfachen Mitteln ein Füllstand ab einer geringen Füllmenge bis zum annähernd gefüllten Fluidsammelbehälter detektiert werden kann.

Diese Aufgabe lösen eine medizinische Saugpumpe mit den Merkmalen des Anspruchs 1 sowie ein Fluidsammelbehälter mit den Merkmalen des Anspruchs 7.

Die erfindungsgemässe medizinische Saugpumpe zum Absaugen von Körperfluid weist ein Saugpumpengehäuse, einen lösbar am Gehäuse gehaltenen Fluidsammelbehälter sowie mindestens einen Füllstandsensor zur Detektion eines Füllstandes des Fluidsammelbehälters auf. Der Fluidsammelbehälter weist eine erste Seitenwand und mindestens eine weitere Seitenwand auf, welche gemeinsam mindestens einen Innenraum zur Aufnahme des abgesaugten Fluids begrenzen, wobei der Innenraum eine mit Fluid befüllbare Höhe aufweist. Der mindestens eine Füllstandsensor erstreckt sich mindestens annähernd parallel zur ersten Seitenwand über mindestens einen Teilbereich der befüllbaren Höhe. Die erste Seitenwand ist mindestens abschnittsweise plan ausgebildet und der mindestens eine Innenraum verjüngt sich mindestens über einen Teilbereich der befüllbaren Höhe im Querschnitt in vertikaler Richtung von oben nach unten.

"Mindestens abschnittsweise" bedeutet in diesem Zusammenhang von Auge erkennbar und nicht infinitesimal.

In einer bevorzugten Ausführungsform erstreckt sich der Füllstandsensor parallel zur ersten Seitenwand und/oder die erste Seitenwand ist durchgehend oder stufenweise plan ausgebildet. Vorzugsweise erstrecken sich diese Stufen in horizontaler Richtung.

Dank der Verjüngung des Querschnitts des Innenraums im unteren Bereich des Behälters, d.h. dort, wo der Behälter als erstes befüllt wird, steigt der Füllstand bei geringer Füllmenge schneller an als bei einer grösseren Füllmenge. Dadurch ist eine Füllmengen-Veränderung im untersten Bereich des Behälters mit einer grösseren Veränderung der Füllhöhe verbunden als im oberen Bereich. Die Messgenauigkeit lässt sich so im Vergleich zu einem Behälter mit gleich bleibendem Querschnitt massiv erhöhen, ohne dass der Sensor an sich geändert werden muss.

Vorzugsweise verläuft die erste Seitenwand in vertikaler Richtung. Dies vereinfacht die Berechnung der Füllstandmenge und gegebenenfalls der Füllgeschwindigkeit oder Füllrate basierend auf dem Sensorsignal.

Vorzugsweise ist die erste Seitenwand füllstandsensorseitig oder auf einer dem mindestens einen Füllstand abgewandten Seite des Fluidsammelbehälters angeordnet. Auch dies erleichtert die Berechnung und erhöht die Messgenauigkeit insgesamt.

Vorzugsweise weist der mindestens eine Füllstandsensor eine Form auf, welche der Form der ersten Seitenwand entspricht, so dass die erste Seitenwand über die gesamte Länge des mindestens einen Füllstandsensors einen gleichbleibenden Abstand zum mindestens einen Füllstandsensor aufweist oder über die gesamte Länge des mindestens einen Füllstandsensors an ihm anliegt. Dadurch ist ein gleichbleibendes Sensorsignal gewährleistet.

Vorzugsweise ist der mindestens eine Füllstandsensor am Saugpumpengehäuse angeordnet. Vorzugsweise ist der mindestens eine Füllstandsensor ein kapazitiver Füllstandsensor. Beispiele derartiger Saugpumpengehäuse und derartiger Sensoren sind in WO 2011/054118 und WO 2012/097462 offenbart. Die Offenbarung dieser zwei Publikationen wird hiermit in diesen Text integriert.

Insbesondere kann der Fluidsammelbehälter ein- und ausschwenkbar am Saugpumpengehäuse gehalten sein, wobei er vollständig lösbar und vom Saugpumpengehäuse entfernbar sein kann.

Ist der Füllstandsensor in oder an einer Wand des Saugpumpengehäuses angeordnet, so weist diese Wand vorzugsweise ein Form auf, welche der Form der ersten Seitenwand entspricht, so dass die erste Seitenwand über die gesamte Länge des mindestens einen Füllstandsensors einen gleichbleibenden Abstand zur Wand des Saugpumpengehäuses aufweist oder über die gesamte Länge des mindestens einen Füllstandsensors an der Wand des Saugpumpengehäuses anliegt.

Alternativ kann ein derartiger oder ein anderer Füllstandsensor auch am Fluidsammelbehälter angeordnet sein bzw. in einer seiner Wände, insbesondere in die erste Seitenwand, integriert sein. Auch hier folgt die Form des Füllstandsensors vorzugsweise derjenigen der Oberfläche der ersten Seitenwand des Fluidsammelbehälters, welche der Innenseite des Behälters zugewandt ist.

Der erfindungsgemässe zugehörige Fluidsammelbehälter weist eine erste Seitenwand und mindestens eine weitere Seitenwand auf, welche gemeinsam mindestens einen Innenraum zur Aufnahme des abgesaugten Fluids begrenzen, wobei der Innenraum eine mit Fluid befüllbare Höhe aufweist. Der Fluidsammelbehälter weist ferner mindestens einen Sauganschluss und mindestens einen Fluidanschluss auf, welche eine Verbindung zum Innenraum ermöglichen. Die erste Seitenwand ist mindestens abschnittsweise plan ausgebildet und der mindestens eine Innenraum verjüngt sich mindestens über einen Teilbereich der befüllbaren Höhe im Querschnitt in vertikaler Richtung von oben nach unten.

Die Seitenwände umgeben den Innenraum. Je nachdem, wie die Seitenwände ausgebildet sind, ist noch ein Boden vorhanden oder sie grenzen so aneinander, dass kein Boden benötigt ist, um den Behälter unten abzuschliessen. Vorzugsweise ist auch eine obere Wand vorhanden. Vorzugsweise umschliessen die Seitenwände, gegebenenfalls gemeinsam mit dem Boden und der Wand, den Innenraum des Behälters so, dass ein bis auf einige Verbindungsöffnungen geschlossener Behälter gebildet ist.

Die erste Seitenwand weist vorzugsweise eine gleichbleibende Dicke auf. Vorzugsweise weisen auch die anderen Wände gleichbleibende Dicken auf. Alle Seitenwände sind vorzugsweise im Wesentlichen durch plane Flächen gebildet.

Die Verjüngung des Innenraums ist vorzugsweise so, dass eine genügende Mess- bzw. Detektionsgenauigkeit erzielt werden kann. Vorzugsweise ist eine untere Querschnittsfläche des Innenraums kleiner oder gleich einem Viertel, vorzugsweise kleiner oder gleich einem Achtel einer oberen Querschnittsfläche des Innenraums.

Um die Berechnung und Messung zu erleichtern, verläuft vorzugsweise nur eine Seitenwand in Bezug auf die vertikale Richtung geneigt und alle anderen Seitenwände verlaufen in vertikaler Richtung. Diese geneigte Seitenwand kann die erste Seitenwand oder eine der anderen Seitenwände sein. Vorzugsweise ist sie eine der anderen Seitenwände. Die Neigung ist vorzugsweise geradlinig, d.h. durch eine plane schräge Ebene gebildet.

In einer bevorzugten Ausführungsform ist eine der mindestens einen weiteren Seitenwand eine zweite Seitenwand, welche wenigstens über den genannten Teilbereich der befüllbaren Höhe in Bezug auf die vertikale Richtung geneigt verläuft. Diese verjüngt somit den Querschnitt.

In einer Ausführungsform weist der Fluidsammelbehälter eine an die erste Seitenwand angrenzende dritte und vierte Seitenwand auf, wobei die zweite Seitenwand der ersten Seitenwand gegenüberliegt und wobei die dritte und vierte Seitenwand plan und in vertikaler Richtung verlaufend ausgebildet sind. In dieser Ausführungsform bilden die dritte und vierte Seitenwand im genannten Teilbereich vorzugsweise je ein spitzwinkliges Dreieck.

In einer anderen Ausführungsform grenzt die zweite Seitenwand an die erste Seitenwand an, wobei eine dritte Seitenwand, welche der zweiten Seitenwand gegenüberliegt, an die erste Seitenwand angrenzt und wobei eine vierte Seitenwand vorhanden ist, welche der ersten Seitenwand gegenüberliegt. Die erste, zweite, dritte und vierte Wand umschliessen einen der mindestens einen Innenräume, wobei die dritte und vierte Wand plan und in vertikaler Richtung verlaufend ausgebildet sind. In dieser Ausführungsform weist der Sammelbehälter vorzugsweise zwei Innenräume auf, welche durch eine Trennwand vollständig voneinander getrennt sind, wobei die Trennwand in vertikaler Richtung verläuft und die dritte Wand bildet.

Vorzugsweise ist der Sauganschluss in der ersten Seitenwand angeordnet. Vorzugsweise ist dies dieselbe Wand, welche dem mindestens einen Füllstandsensor zugewandt und am nächsten ist. Die Saugpumpe ist vorzugsweise eine elektromotorisch betriebene Vakuumpumpe, insbesondere eine Kolbenpumpe oder eine Membranpumpe.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine perspektivische Darstellung einer erfmdungsgemässen Saugpumpe mit einem erfindungsgemässen Fluidsammelbehälter in einer ersten Ausführungsform;
- Figur 2: die Saugpumpe gemäss Figur 1 mit ausgeschwenktem Fluidsammelbehälter;
- Figur 3: eine perspektivische Darstellung einer Variante des Fluidsammelbehälters gemäss Figur 1;
- Figur 4: eine Seitenansicht des Fluidsammelbehälters gemäss Figur 3;
- Figur 5: eine Seitenansicht eines erfindungsgemässen Fluidsammelbehälters in einer zweiten Ausführungsform;
- Figur 6: eine Seitenansicht eines erfindungsgemässen Fluidsammelbehälters in einer dritten Ausführungsform;
- Figur 7: eine perspektivische Darstellung eines erfindungsgemässen Fluidsammelbehälters mit sichtbar dargestelltem Innenraum in einer vierten Ausführungsform;
- Figur 8: einen ersten Längsschnitt durch den Fluidsammelbehälter gemäss Figur 7;
- Figur 9: einen zweiten Längsschnitt durch den Fluidsammelbehälter gemäss Figur 7 und
- Figur 10: einen Querschnitt durch den Fluidsammelbehälter gemäss Figur 7.

### BESCHREIBUNG BEVORZUGTER AUSFÜHRUNGSFORMEN

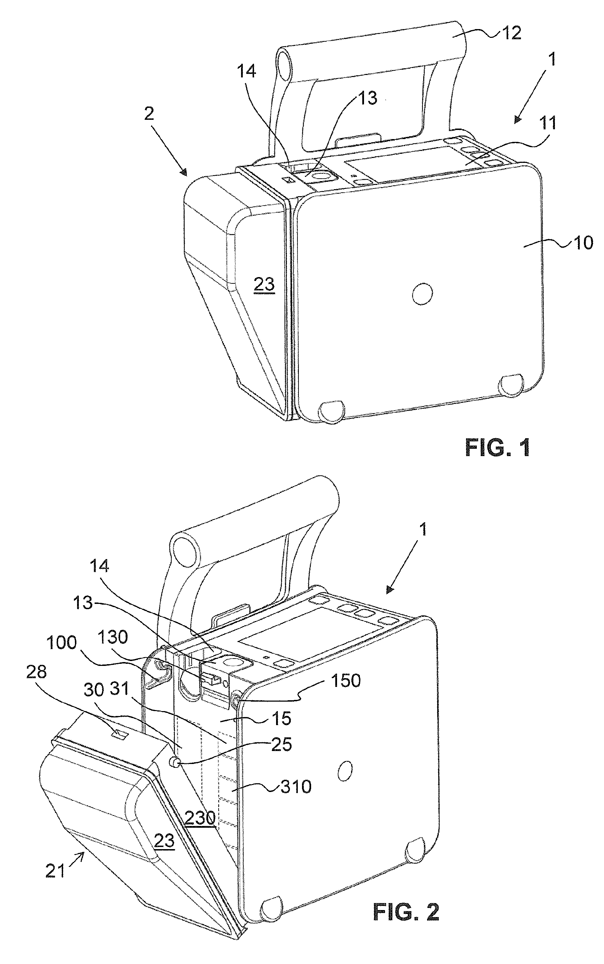

In den Figuren 1 und 2 ist eine Drainagepumpeinheit dargestellt, wie sie im Grundaufbau beispielsweise aus WO 2007/128156 und WO 2008/141471 bekannt ist. Diese Pumpeinheiten dienen zum Absaugen von Körperflüssigkeiten bzw. -fluiden im medizinischen Bereich und sind vorzugsweise tragbar ausgebildet. Das heisst, der Patient kann sich während des Abpumpens frei bewegen.

Die Drainagepumpeinheit weist ein Saugpumpengehäuse 1 mit einem lösbar daran befestigten, vollständig vom Gehäuse 1 entfernbaren Fluidsammelbehälter 2. Das Saugpumpengehäuse 1 und der Fluidsammelbehälter 2 sind vorzugsweise aus Kunststoff gefertigt.

Im Gehäuse 1 sind das Pumpaggregat und eine Steuerelektronik angeordnet. Das Gehäuse ist in diesem Beispiel quaderförmig ausgebildet. Es kann jedoch auch andere Formen aufweisen.

In einer oberen Wand des Gehäuses 1 ist ein Anzeige- und Bedienfeld 11 angeordnet. Ein Handgriff 12 erleichtert den Transport. Vorzugsweise weist das Gehäuse 1 alternativ oder zusätzlich zum Traggriff 12 eine Standfläche oder Standfüsse auf, so dass das Gehäuse 1 in der abgebildeten Lage auf einem Tisch oder einer anderen geeigneten Standfläche aufgestellt werden kann. Nachfolgend angegebene Richtungsangaben sind in dieser stehenden Position des Gehäuses zu verstehen.

Eine in den Figuren sichtbare vordere Wand ist mit dem Bezugszeichen 10 bezeichnet, eine Seitenwand mit 15. Die sichtbare vordere Wand 10 und eine ihr gegenüber liegende, parallel dazu verlaufende Rückwand stehen der Seitenwand 15 vor. Sie bilden eine Aufnahme für den Fluidsammelbehälter 2.

Die obere Wand weist im Bereich der Seitenwand 15 eine Ausnehmung 14 auf. Ferner ist dort auch ein Verriegelungselement 13 vorhanden, welches eine Einrastnase 130 aufweist. Dieses Verriegelungselement 13 dient zur lösbaren Fixierung des Fluidsammelbehälters 2 am Gehäuse 1. Der Fluidsammelbehälter 2 weist hierzu vorzugsweise eine Ausnehmung 28 auf.

Der Fluidsammelbehälter 2 wird vorzugsweise in das Gehäuse 1 eingeschwenkt und rastet in dieser Position ein. Hierzu weisen der vorstehende Teil der vorderen Wand 10 und der Rückwand obere und untere Kulissenführungen 100 oder Einrastöffnungen auf, in welche untere und obere Einrastbolzen 24, 25 des Fluidsammelbehälters 2 eingreifen.

Anstelle von Kulissenführungen, Einrastbolzen und Einrasthaken kann die Befestigung des Sammelbehälters 2 und seine Fixierung auch mit anderen Mitteln erfolgen. Auch die körperliche Gestaltung des Gehäuses 1 und des Sammelbehälters 2 im Bereich ihrer Angrenzung aneinander kann anders ausgebildet sein.

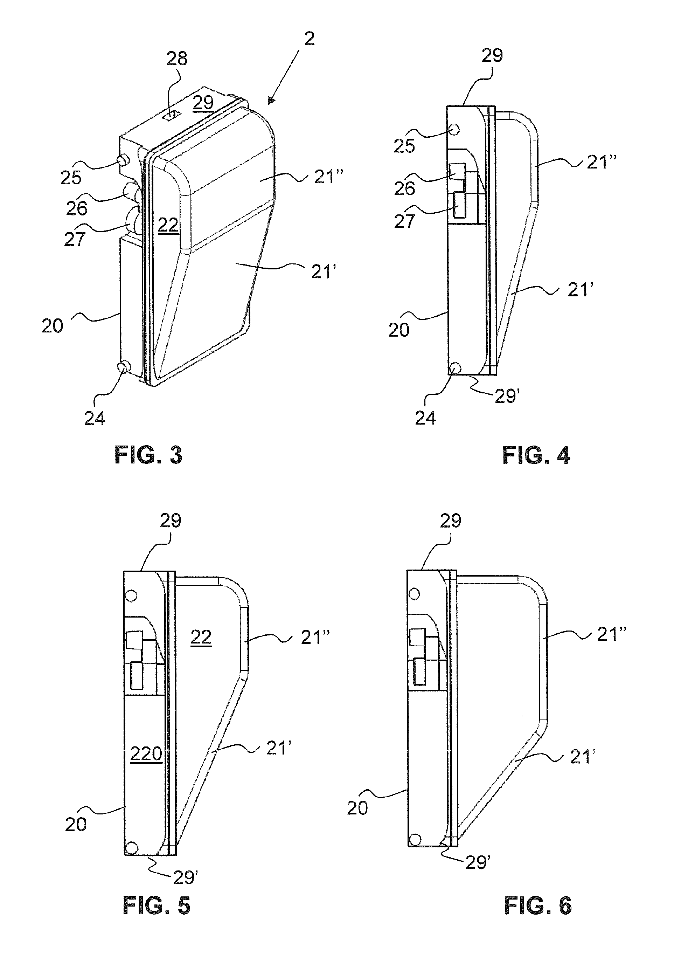

Der Fluidsammelbehälter weist eine erste Seitenwand 20 auf, welche in vertikaler Richtung verläuft und plan ausgebildet ist. Diese Seitenwand 20 ist in den Figuren 3 und 4 gut erkennbar. Die Figuren 3 und 4 zeigen einen Behälter 2, welcher gleich ausgebildet ist wie der in den Figuren 1 und 2 dargestellte Behälter 2. Lediglich die Öffnungen in seiner ersten Seitenwand 20 sind nicht an derselben Stelle angeordnet. So befinden sich im Behälter 2 gemäss den Figuren 1 und 2 ein Drainageanschluss zur Verbindung mit einem patientenseitigen Adapterteil auf einer linken Seite der ersten Seitenwand 20, ein Vakuumanschluss zur Verbindung mit der Saugpumpe auf einer rechten Seite der ersten Seitenwand 20 und eine Entleerungsöffnung zur Entleerung des Behälters 2 ebenfalls auf der rechten Seite. Im Ausführungsbeispiel gemäss den Figuren 3 und 4 befinden sich hingegen der Drainageanschluss auf der rechten Seite und der Vakuumanschluss 26 sowie die Entleerungsöffnung 27 auf der linken Seite. Entsprechend sind auch die dazu passenden Gegenstücke im Pumpengehäuse 1, d.h. ein Vakuumanschluss 150 und die Ausnehmung 14 zur Aufnahme eines patientenseitigen Adapterteils, anders als in den Figuren 1 und 2 dargestellt angeordnet. Im Folgenden wird auf die Figuren 3 und 4 Bezug genommen, wenn der Behälter gemäss den Figuren 1 und 2 beschrieben wird.

Mit der oben erwähnten ersten Seitenwand 20 liegt der Fluidsammelbehälter 2 an der Seitenwand 15 des Saugpumpengehäuses 1 an, wobei ein kleiner Luftspalt vorhanden sein kann. Die Seitenwand 15 des Gehäuses 1 ist ebenfalls plan ausgebildet und verläuft in vertikaler Richtung. Dabei ist je ein rechteckförmiger Abschnitt 220, 230 von zwei gegenüberliegenden, an die erste Seitenwand 20 angrenzenden Seitenwänden 22, 23 zwischen der vorstehenden vorderen Wand 10 und der Rückwand des Gehäuses 1 aufgenommen.

In der Seitenwand 15 des Gehäuses 1 ist der gehäuseseitige Vakuumanschluss 150 angeordnet. Vorzugsweise ist er in einem oberen Bereich der Seitenwand 15 angeordnet. Der Vakuumanschluss 150 wird beim Einschwenken des Behälters 2 mit dem in den Figuren 3 und 4 dargestellten Vakuumanschluss 26 des Behälters 2 verbunden.

In die Ausnehmung 14 lässt sich ein Adapterteil eines patientenseitigen Drainageschlauchs einstecken und durch Einschwenken des Behälters 2 mit dem in den Figuren nicht sichtbaren Drainageanschluss des Behälters 2 verbinden. Dies ist beispielsweise aus WO 2008/141471 bekannt.

Die Bezugsziffer 27 zeigt auf die Entleerungsöffnung, welche bei Gebrauch des Behälters 2 verschlossen ist und erst zur Entleerung des gefüllten und aus dem Pumpengehäuse 1 entfernten Behälters 2 geöffnet wird.

Über den Vakuumanschluss 150 wird mittels der Saugpumpe ein Unterdruck im Behälter 2 erzeugt. Dank diesem Unterdruck wird ein Fluid oder Sekret aus der Kavität des Patienten durch einen Drainageschlauch und durch den Drainageanschluss in den Behälter 2 gesaugt und dort gesammelt.

Die Saugpumpe umfasst ferner mindestens einen kapazitiven Füllstandsensor, mittels welchem der Füllstand im Fluidsammelbehälter 2 feststellbar ist. Der Füllstandsensor ist in diesem Beispiel in der Gehäuse-Seitenwand 15, welche dem Fluidsammelbehälter 2 zugewandt ist, angeordnet.

Der Sensor weist mindestens zwei, hier genau zwei Elektroden 30, 31 auf, welche beabstandet zueinander angeordnet sind. Eine erste Elektrode 30 ist einstückig ausgebildet und bildet einen Sender. Eine zweite Elektrode 31 ist segmentiert ausgebildet und bildet einen Empfänger zum Empfang des vom Sender ausgesendeten Signals. Es wird ein Ladungstransfer von Sender- zur Empfängerelektrode ermittelt. Die zwei Elektroden 30, 31 verlaufen parallel beabstandet zueinander. In diesem Beispiel sind sie in einer gemeinsamen Ebene angeordnet. Ihre Sender- bzw. Empfängerflächen sind dabei in dieselbe Richtung gerichtet, nämlich zum Fluidsammelbehälter 2 hin. Dieser Füllstandsensor ist in WO 2012/097462 beschrieben. Alternativ lässt sich auch ein anderer Füllstandsensor einsetzen, vorzugsweise ein kapazitiver Füllstandsensor. Der Füllstandsensor, hier gebildet durch die zwei Elektroden 30, 31, ist bandförmig ausgebildet und erstreckt sich in vertikaler Richtung über einen gewünschten Messbereich des Behälters 2, vorzugsweise über die zu messende bzw. zu detektierende Füllhöhe.

Wie in den Figuren 1 bis 4 erkennbar ist, weist der erfmdungsgemässe Behälter 2 einen sich nach unten verjüngenden Querschnitt auf. Hierzu weisen die dritte und die vierte Seitenwand 22, 23 einen Abschnitt auf, welcher als spitzwinkliges Dreieck ausgebildet ist. Entsprechend ist die der ersten Seitenwand 20 gegenüberliegende zweite Seitenwand 21 geneigt ausgebildet. Sie weist vorzugsweise einen zur vertikalen Richtung hin geneigten Abschnitt 21' und einen vertikal verlaufenden mittleren Abschnitt 21" auf. Ein oberer Abschnitt ist vorzugsweise gebogen ausgebildet und geht in eine obere Wand 29 über. Ein Boden 29' des Behälters 2 ist im Vergleich zur oberen Wand 29 entsprechend kleiner ausgebildet.

Entsprechend zur Form dieser Wände ist der Innenraum des Behälters 2 ausgebildet. Vorzugsweise weisen die Wände deshalb eine gleichbleibende Dicke auf.

In den Figuren 5 und 6 sind ein zweites und ein drittes Ausführungsbeispiel des Behälters 2 dargestellt. Der Innenraum weist ein grösseres Volumen auf als der Behälter gemäss den Figuren 3 und 4. Diese Behälter 2 lassen sich jedoch in dasselbe Gehäuse 1 gemäss den Figuren 1 und 2 einschwenken und halten. Wie durch den Vergleich der Figuren 4 bis 6 erkennbar ist, unterscheiden sie sich lediglich im Neigungswinkel und der Länge des unteren Abschnitts 21' der zweiten Seitenwand 21 und somit in der Form der dritten und vierten Seitenwand 22, 23 voneinander.

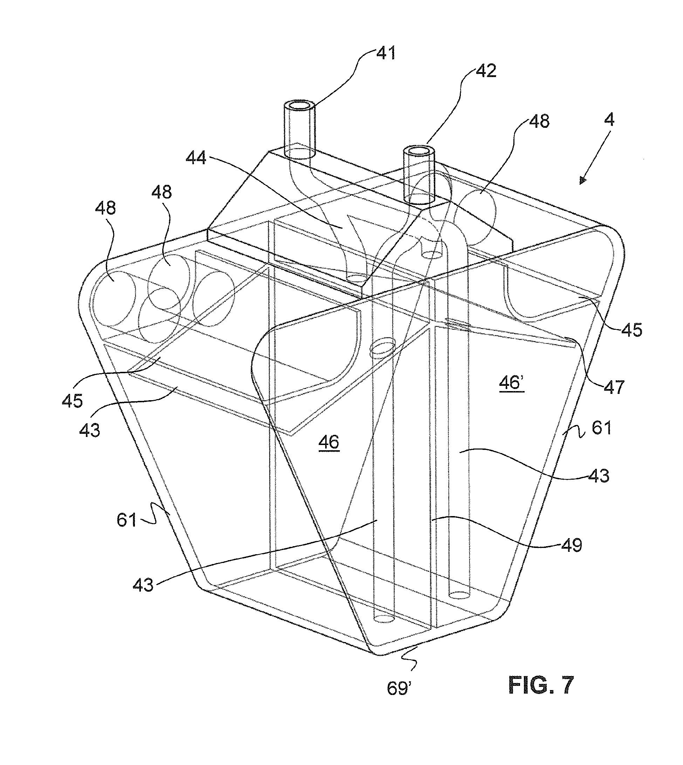

In den Figuren 7 bis 10 ist ein viertes Ausführungsbeispiel eines erfindungsgemässen Fluidsammelbehälters 4 dargestellt. Dieser Behälter lässt sich in einem anders geformten Saugpumpengehäuse halten. Es ist jedoch auch wieder möglich, ihn schwenkbar, lösbar und vollständig entfernbar zu halten, beispielsweise indem er mit entsprechenden Bolzen versehen wird. Dieser Behälter eignet sich insbesondere zur Verwendung in einem Autotransfusionsgerät zur Aufnahme und Weiterleitung des Bluts, beispielsweise zu einer Herz-Lungen-Maschine.

Dieser Fluidsammelbehälter 4 weist zwei vollständig voneinander getrennte Kammern 46, 46' und somit zwei voneinander unabhängige Innenräume auf. Entsprechend sind zwei Füllstandsensoren vorhanden, welche hier nicht dargestellt sind. Es lassen sich beispielsweise die oben beschriebenen Füllstandsensoren, insbesondere zwei Elektrodenpaare mit je zwei bandförmigen, zueinander beabstandeten Elektroden, verwenden.

Die Kammern dienen bei Verwendung in einem Autotransfusionsgerät zum Sammeln von Blut. Deshalb wird im Folgenden von Blut gesprochen, wobei sich der Behälter auch zum Sammeln eines anderen Körperfluids eignet.

Der Behälter 4 ist vorzugsweise einstückig ausgebildet. Vorzugsweise besteht er aus einem Kunststoff. Vorzugsweise ist er wie auch die oben bereits erwähnten Behälter 2 durchsichtig und vorzugsweise als Einwegprodukt ausgebildet und wird nach Verwendung entsorgt.

Der Behälter 4 weist auf seiner Oberseite einen patientenseitigen Anschluss 41 und einen weiterleitungsseitigen Anschluss 42 auf. Der Behälter 4 ist ansonsten spiegelsymmetrisch aufgebaut. Vom patientenseitigen Anschluss 41 führt eine sich verzweigende, eine Y-Form aufweisende Verbindungsleitung 44 in den Behälterinnenraum. Von einem weiterleitungsseitigen Anschluss 42 führt eine Y-Verzweigung in zwei Steigrohre 43, welche sich bis in den unteren Bereich des Innenraums des Behälters erstrecken.

Der Innenraum des Behälters 4 ist in zwei Bereiche unterteilt, welche vorzugsweise vollständig voneinander getrennt sind. Die Bereiche befinden sich in diesem Beispiel nebeneinander und sind durch eine Trennwand 49 voneinander getrennt. Je eine der genannten Verbindungsleitungen 44 und je eines der genannten Steigrohre 43 führen in je einen der Bereiche. Im Folgenden wird ein einzelner Bereich beschrieben. Der andere Bereich ist identisch aufgebaut.

Der Bereich wird im Wesentlichen durch eine Blutsammelkammer 46 gebildet, welche in ihrem oberen Bereich von einer Schrägrippe 47 begrenzt ist. Diese Schrägrippe 47 erstreckt sich von der Trennwand 49 beginnend zur gegenüberliegenden Seite hin nach unten, wobei sie sich fast, jedoch nicht ganz vollständig über die gesamte Breite der Kammer erstreckt. In der dazu senkrechten Richtung erstreckt sich die Schrägrippe 47 über die gesamte Länge des Bereichs bzw. der Kammer 46. Die Schrägrippe 47 ist vom Steigrohr 43 durchsetzt.

Im Bereich oberhalb der Schrägrippe 47 ist die sich über die gesamte Länge des Bereichs hin erstreckende und nach oben gebogene Trennrippe 45 angeordnet, welche einen pumpenseitigen Bereich definiert. Dieser Bereich ist lediglich durch einen schmalen oberen Spalt mit dem restlichen Innenraum verbunden. In diesem Bereich sind eine erste und eine zweite Anschlussöffnung 40, 40' angeordnet, welche zur Verbindung mit einer Vakuumleitung bzw. einer Überdruckleitung der Saugpumpe dienen. Diese Anschlussöffnungen 40, 40' sind mit Filtern 48 versehen. Diese Filter 48 sind im Stand der Technik bekannt. Es können auch noch andere Filter an anderen Stellen vorhanden sein, wie dies ebenfalls aus dem Stand der Technik bekannt ist. Diese Filter dienen zum Schutz des Geräts.

Die Trennrippe 45 verhindert, dass Blut zu den Filtern spritzen kann und diese vorzeitig verschliessen kann. Zudem bildet sie einen weiteren Schutz für die Pumpen. Die Schrägrippe 47 verhindert ein Zurückschwappen des abgesaugten Bluts in die Saugleitung und ermöglicht zudem, dass abgesaugtes Blut über die Schrägrippe 47 nach unten in die Sammelkammer 46 fliessen kann.

Die Kammer 46 weist einen sich nach unten verjüngenden Querschnitt auf. Vorzugsweise beträgt die Fläche im untersten Bereich der Kammer 46 maximal die Hälfte der Fläche im oberen Bereich, d.h. im Bereich knapp unterhalb des unteren Endes der Schrägrippe 47. Dies ermöglicht eine relativ genaue Füllstandmessung selbst bei geringen Füllmengen, insbesondere wenn hierfür kapazitive Füllstandsensoren eingesetzt werden.

Vorzugsweise weist der Blutsammelbehälter 4 deshalb eine vertikal verlaufende, plane und trapezförmige erste Seitenwand 60, zwei einander gegenüberliegende, schräg verlaufende zweite Seitenwände 61, eine zwischen den zwei zweiten Seitenwänden 61 vertikal verlaufende dritte Seitenwand in Form der Trennwand 49, sowie eine der ersten Seitenwand 60 gegenüberliegende, planparallel zu dieser verlaufende und ebenfalls trapezförmige vierte Seitenwand 63 auf. Eine obere Wand trägt das Bezugszeichen 69, ein unterer Boden das Bezugszeichen 69'.

Die Füllstandsensoren sind vorzugsweise benachbart zur ersten Seitenwand 60 bei den entsprechenden Kammern 46, 46' angeordnet. Auch sind die Sensoren vorzugsweise am Gehäuse angeordnet und verlaufen in ihrer Form der ersten Seitenwand 60 entsprechend. Sie können jedoch auch an oder in der ersten Seitenwand angeordnet sein.

Die zwei Kammern 46 des Behälters 4 lassen sich abwechslungsweise befüllen und entleeren. D.h. wird eine Kammer befüllt, so lässt sich die andere gleichzeitig entleeren. Dabei wird spätestens dann entleert, wenn der entsprechende Füllstandsensor einer Steuereinheit des Geräts meldet, dass die entsprechende Kammer voll ist.

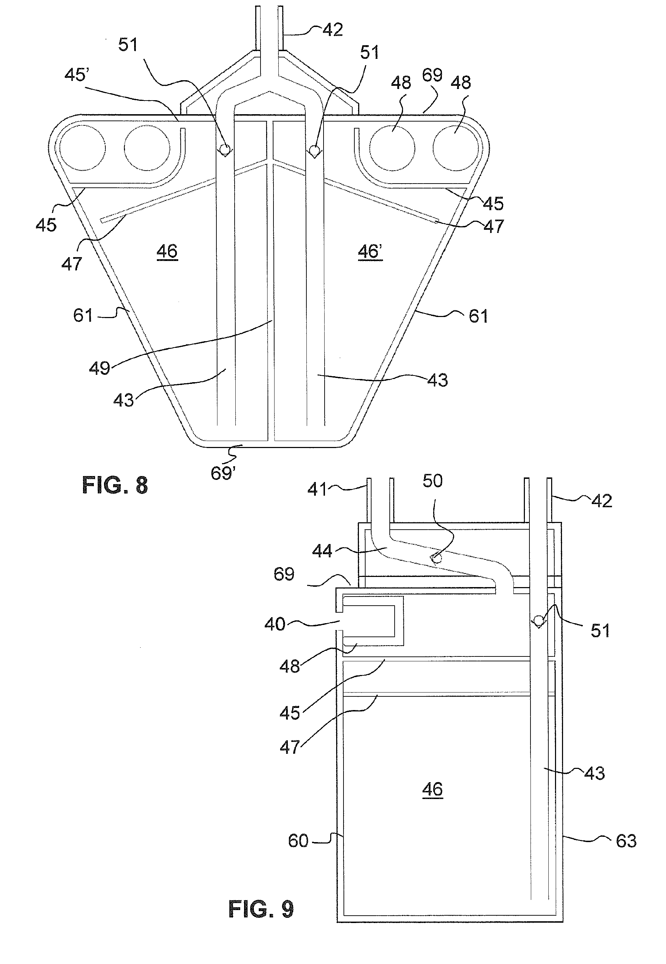

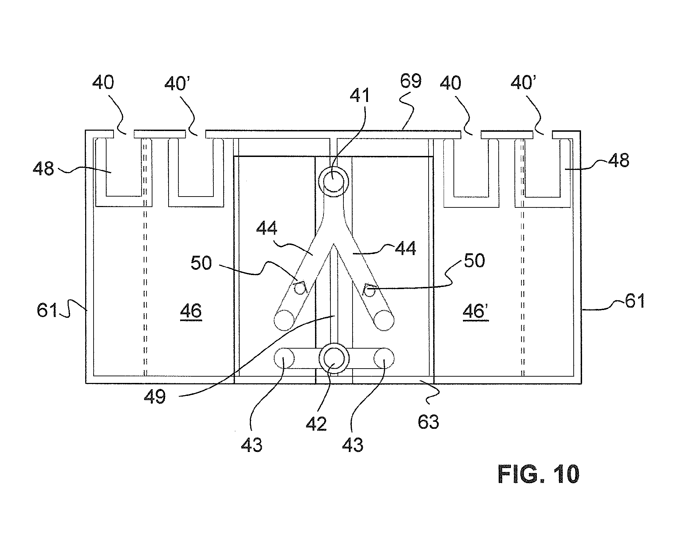

Entsprechend sind Einwegventile 50 vorhanden, welche die Verbindung zwischen der Saugleitung und der Sammelkammer 46 öffnen und verschliessen. Diese können über die Steuerung gesteuert sein oder bei einem vorgegebenen Druck schliessen. Sie können, wie in den Figuren 9 und 10 dargestellt, Teil des Blutsammelbehälters 4 sein oder sie können auch im Bereich der Saugleitung bzw. beim patientenseitigen Anschluss angeordnet sein.

Es sind ferner Einwegventile 51 vorhanden, welche die Verbindung zwischen der Sammelkammer 46 und der Weiterleitung öffnen und verschliessen. Diese können ebenfalls über die Steuerung gesteuert sein oder bei einem vorgegebenen Druck schliessen. Sie können, wie in den Figuren 8 und 9 dargestellt, Teil des Blutsammelbehälters 4 sein oder sie können auch im Bereich der Weiterleitung bzw. beim weiterleitungsseitigen Anschluss 42 angeordnet sein.

Die Einwegventile können beispielsweise einfache Rückschlagventile sein oder sie können ebenfalls über die Steuereinheit gesteuert sein.

Die erfindungsgemässe Saugpumpe und der erfindungsgemässe Fluidsammelbehälter ermöglichen eine relativ genaue Detektion eines Füllstandes bereits bei geringer Füllstandhöhe.

**BEZUGSZEICHENLISTE**

| | | | |
|---|---|---|---|
| 1 | Gehäuse | | |
| 10 | vordere Wand | 30 | erste Elektrode |
| 100 | Kulissenführung | 31 | zweite Elektrode |
| 11 | Anzeige- und Bedienfeld | 310 | Elektrodensegment |
| 12 | Traggriff | | |
| 13 | Verriegelungselement | 4 | Fluidsammelbehälter |
| 130 | Einrastnase | 40 | erste Anschlussöffnung |
| 14 | Ausnehmung | 40' | zweite Anschlussöffnung |
| 15 | Seitenwand | 41 | patientenseitiger Anschluss |
| 150 | Vakuumanschluss | 42 | weiterleitungsseitiger Anschluss |
| 2 | Fluidsammelbehälter | 43 | Steigleitung |
| 20 | erste Seitenwand | 44 | Verbindungsleitung |
| 21 | zweite Seitenwand | 45 | Trennrippe |
| 21' | unterer Bereich der zweiten Wand | 45' | Spalt |
| | | 46 | erste Sammelkammer |
| 21" | mittlerer Bereich der zweiten Seitenwand | 46' | zweite Sammelkammer |
| | | 47 | Schrägrippe |
| 22 | dritte Seitenwand | 48 | Filter |
| 220 | Abschnitt | 49 | Trennwand |
| 23 | vierte Seitenwand | | |
| 230 | Abschnitt | 50 | patientenseitiges Ventil |
| 24 | unterer Befestigungsbolzen | 51 | weiterleitungsseitiges Ventil |
| 25 | oberer Befestigungsbolzen | | |
| 26 | Vakuumanschluss | 60 | erste Seitenwand |
| 27 | Entleerungsöffnung | 61 | zweite Seitenwand |
| 28 | Ausnehmung | 63 | vierte Seitenwand |
| 29 | obere Wand | 69 | obere Wand |
| 29' | Boden | 69' | Boden |

## Patentansprüche

1. Medizinische Saugpumpe zum Absaugen von Körperfluid, wobei die Saugpumpe ein Saugpumpengehäuse (1), einen lösbar am Gehäuse (1) gehaltenen Fluidsammelbehälter (2, 4) sowie mindestens einen Füllstandsensor (30, 31) zur Detektion eines Füllstandes des Fluidsammelbehälters (2, 4) aufweist,
wobei der Fluidsammelbehälter (2) eine erste Seitenwand (20, 60) und mindestens eine weitere Seitenwand (21, 22, 23; 61, 49, 63) aufweist, welche gemeinsam mindestens einen Innenraum zur Aufnahme des abgesaugten Fluids begrenzen, wobei der Innenraum eine mit Fluid befüllbare Höhe aufweist,
wobei sich der mindestens eine Füllstandsensor (30, 31) mindestens annähernd parallel zur ersten Seitenwand (20, 60) über mindestens einen Teilbereich der befüllbaren Höhe erstreckt,
**dadurch gekennzeichnet,**
**dass** die erste Seitenwand (20, 60) mindestens abschnittsweise plan ausgebildet ist und
**dass** sich der mindestens eine Innenraum mindestens über einen Teilbereich der befüllbaren Höhe im Querschnitt in vertikaler Richtung von oben nach unten verjüngt.

2. Saugpumpe nach Anspruch 1, wobei die erste Seitenwand (20, 60) in vertikaler Richtung verläuft.

3. Saugpumpe nach einem der Ansprüche 1 oder 2, wobei die erste Seitenwand (20, 60) füllstandsensorseitig oder auf einer dem mindestens einen Füllstand abgewandten Seite des Fluidsammelbehälters (2, 4) angeordnet ist.

4. Saugpumpe nach einem der Ansprüche 1 bis 3, wobei der mindestens eine Füllstandsensor (30, 31) eine Form aufweist, welche der Form der ersten Seitenwand (20, 60) entspricht, so dass die erste Seitenwand (20, 60) über die gesamte Länge des mindestens einen Füllstandsensors (30, 31) einen gleichbleibenden Abstand zum mindestens einen Füllstandsensor (30, 31) aufweist oder über die gesamte Länge des mindestens einen Füllstandsensors (30, 31) an ihm anliegt.

5. Saugpumpe nach einem der Ansprüche 1 bis 4, wobei der mindestens eine Füllstandsensor (30, 31) am Saugpumpengehäuse (1) angeordnet ist.

6. Saugpumpe nach einem der Ansprüche 1 bis 5, wobei der mindestens eine Füllstandsensor (30, 31) ein kapazitiver Füllstandsensor ist.

7. Fluidsammelbehälter einer medizinischen Saugpumpe gemäss einem der Ansprüche 1 bis 6, wobei der Fluidsammelbehälter (2, 4) eine erste Seitenwand (20, 60) und mindestens eine weitere Seitenwand (21, 22, 23; 61, 49, 63) aufweist, welche gemeinsam mindestens einen Innenraum zur Aufnahme des abgesaugten Fluids begrenzen, wobei der Innenraum eine mit Fluid befüllbare Höhe aufweist,
wobei der Fluidsammelbehälter (2, 4) ferner mindestens einen Sauganschluss (40) und mindestens einen Fluidanschluss (41) aufweist, welche eine Verbindung zum Innenraum ermöglichen,
**dadurch gekennzeichnet,**
**dass** die erste Seitenwand (20, 60) mindestens abschnittsweise plan ausgebildet ist und
**dass** sich der mindestens eine Innenraum mindestens über einen Teilbereich der befüllbaren Höhe im Querschnitt in vertikaler Richtung von oben nach unten verjüngt.

8. Fluidsammelbehälter nach Anspruch 7, wobei alle Seitenwände im Wesentlichen durch plane Flächen gebildet sind.

9. Fluidsammelbehälter nach Anspruch 8, wobei nur eine der Seitenwände (21, 61) in Bezug auf die vertikale Richtung geneigt verläuft und alle anderen Seitenwände (20, 22, 23; 60, 49, 63) in vertikaler Richtung verlaufen.

10. Fluidsammelbehälter nach einem der Ansprüche 7 bis 9, wobei eine der mindestens einen weiteren Seitenwand eine zweite Seitenwand (21, 61) ist, welche wenigstens über den genannten Teilbereich der befüllbaren Höhe in Bezug auf die vertikale Richtung geneigt verläuft.

11. Fluidsammelbehälter nach Anspruch 10, wobei der Fluidsammelbehälter (2) eine an die erste Seitenwand (20) angrenzende dritte und vierte Seitenwand (22, 23) aufweist und wobei die zweite Seitenwand (21) der ersten Seitenwand (20) gegenüberliegt, wobei die dritte und vierte Seitenwand (22, 23) plan und in vertikaler Richtung verlaufend ausgebildet sind.

12. Fluidsammelbehälter nach Anspruch 11, wobei die dritte und vierte Seitenwand (22, 23) im genannten Teilbereich je ein spitzwinkliges Dreieck bilden.

13. Fluidsammelbehälter nach Anspruch 10, wobei die zweite Seitenwand (61) an die erste Seitenwand (60) angrenzt und wobei eine dritte Seitenwand (49), welche der zweiten Seitenwand (61) gegenüberliegt, an die erste Seitenwand (60) angrenzt und wobei eine vierte Seitenwand (63) vorhanden ist, welche der ersten Seitenwand (60) gegenüberliegt, wobei die erste, zweite, dritte und vierte Wand (60, 61, 49, 63) einen der mindestens einen Innenräume umschliessen, wobei die dritte und vierte Wand (49, 63) plan und in vertikaler Richtung verlaufend ausgebildet sind.

14. Fluidsammelbehälter nach Anspruch 13, wobei der Sammelbehälter (4) zwei Innenräume aufweist, welche durch eine Trennwand (49) vollständig voneinander getrennt sind, wobei die Trennwand (49) in vertikaler Richtung verläuft und die dritte Wand bildet.

15. Fluidsammelbehälter nach einem der Ansprüche 7 bis 14, wobei der Sauganschluss (40, 40') in der ersten Seitenwand (60) angeordnet ist.
